# EUROPEAN PATENT APPLICATION

(11) **EP 1 498 067 A1**
(43) Date of publication of application: **19.01.2005**
(21) Application number: 03720907.9
(22) Date of filing: 16.04.2003
(51) Int. Cl.: A61B 5/00, A61M 5/00, G06F 17/60

(54) **DOSAGE DETERMINATION SUPPORTING DEVICE, INJECTOR, AND HEALTH MANAGEMENT SUPPORTING SYSTEM**

(30) Priority: 25.04.2002 JP 2002123976
(71) Applicant: MATSUSHITA ELECTRIC INDUSTRIAL CO., LTD., Kadoma-shi, Osaka 571-8501 (JP)
(72) Inventor: WATANABE, Motokazu, Toyonaka-shi, Osaka 560-0025 (JP); YOSHIOKA, Toshihiko, Hirakata-shi, Osaka 573-0035 (JP)
(74) Representative: Pautex Schneider, Nicole
(86) International application number: PCT/JP2003/004807
(87) International publication number: WO 2003/090614

(57) **Abstract**

A dosage determination supporting apparatus, which is able to precisely determine a dosage in accordance with the health condition of a user, is provided with: a sensor (28) for measuring the blood sugar level obtained from the blood of the user; a memory (48) for storing an operation table showing a correspondence between the blood sugar level and the amount of insulin; a CPU (50) for calculating the amount of insulin corresponding to the blood sugar level, with reference to the operation table stored in the memory (48); a displaying unit (34) for displaying the amount of insulin; and a voice processing unit (52) for performing the voice processing on the amount of insulin and outputting the voice through a speaker (32).

## Description

### Technical Field

The present invention relates to a dosage determination supporting apparatus, a syringe, and a health-care supporting system, and in particular, relates to a dosage determination supporting apparatus, a syringe, and a health-care supporting system that can automatically determine a dosage in accordance with physical condition of a user.

### Background Art

Diabetes is a disease that is caused by a metabolic aberration resulted from an insufficiency of insulin secreted from the pancreas. As a treatment for this diabetes, insulin therapy is now widely used for controlling blood sugar level by supplementing insulin through an injection.

In many cases, a patient who receives the insulin therapy measures his/her glucose concentration in the blood (henceforth, referred to as the "blood sugar level") before an insulin injection in order to adjust the amount of insulin to be injected or to consider it as a barometer of a therapeutic effect of insulin. Therefore, on the occasion of insulin injection, not only an insulin syringe, but also an equipment for measuring the blood sugar level (henceforth, referred to as the "blood sugar meter") is required. Since the patient needs to inject insulin even when he/she is away home, it is desired that the portability of these insulin syringe and blood sugar meter should be improved. Moreover, to achieve a series of actuations from the measurement of the blood sugar level to the insulin injection, the blood sugar level has to be measured using the blood sugar meter, which is then switched over the insulin syringe for the injection, and this is complicated. With this being the situation, it has been desired to ease the series of actuations especially for elderly people and patients whose function of fingers has deteriorated due to diabetic complication.

As a conventional insulin syringe that satisfies such a requirement, an insulin syringe equipped with a blood sugar meter is disclosed in the pamphlet of International Publication WO95/24233.

This insulin syringe is formed integrally with a blood sugar meter, with its usability being improved for the patients. Also, this insulin syringe is capable of externally outputting a measurement result of the blood sugar level and the amount of insulin to be injected.

However, the conventional insulin syringe is only a combination of a blood sugar meter and an insulin syringe. For this reason, in order to inject insulin, the patient has to obtain the amount of insulin to be injected by referring to a conversion table or the like on the basis of the measurement result of the blood sugar level. However, for a patient whose vision has deteriorated due to diabetic complication, this activity is difficult and carries a problem of the risk that the incorrect amount of insulin may be obtained.

Moreover, there is also a problem that setting the obtained amount of insulin to the syringe is difficult for a patient whose vision has weakened and a patient whose function of fingers has deteriorated.

Such problems are not limited to diabetics, and these are important problems for all the patients who need the administration of medication.

With this being the situation, the present invention is invented to solve the above-mentioned problems and its object is to provide a dosage determination supporting apparatus that can precisely determine a dosage in accordance with the physical condition of the user.

Moreover, its object is also to provide a syringe that can accurately inject the dosage of medicine set in accordance with the physical condition of the user.

Furthermore, its object is also to provide a health-care supporting system that can precisely determine a dosage in accordance with the physical condition of the user.

### Disclosure of Invention

A dosage determination supporting apparatus associated with an aspect of the present invention is a dosage determination supporting apparatus which supports a of a dosage of a medicine, being provided with: a measuring unit operable to measure biological information obtained from one of inside and surface of a user's body; a dosage calculating unit operable to calculate a dosage on the basis of the biological information; and a notifying unit operable to notify the user of the dosage calculated by the dosage calculating unit.

According to this construction, the biological information is measured by the measuring unit, the dosage is calculated on the basis of the biological information, and the user is notified of it. On account of this, the physical condition of the user is perceived from the biological information, and the dosage corresponding to it is precisely determined. Here, the biological information refers to a sample characteristic (blood sugar level, cholesterol reading, etc.) of the blood or saliva, for example.

Preferably, the dosage calculating unit includes: a correspondence memory operable to memorize a correspondence between the biological information and the dosage; and a calculating unit operable to calculate the dosage corresponding to the biological information, with reference to the correspondence.

More preferably, the dosage determination supporting apparatus may be further provided with: a communication controlling unit, which is connected to an external terminal via a network, operable to control a communication with the external terminal; and a correspondence rewriting unit operable to receive an external input via the communication controlling unit and to rewrite contents of the correspondence stored in the correspondence memory on the basis of the external input.

According to this construction, the correspondence can be rewritten externally. Owing to this, the user can always determine the optimal dosage corresponding to his/her physical condition.

More preferably, the dosage determination supporting apparatus may be further provided with an external input authenticating unit operable to authenticate a person who inputs the external input, wherein the correspondence rewriting unit rewrites the contents of the correspondence stored in the correspondence memory on the basis of the external input only when the person is approved.

According to this construction, the correspondence cannot be rewritten by anybody except for those who are previously approved, such as a physician. On account of this, the user can use the dosage determination supporting apparatus with a sense of security.

Preferably, the dosage determination supporting apparatus may be further provided with: a dosage memory operable to memorize the dosage; a dosage storing unit operable to store the calculated dosage together with a calculation time of day into the dosage memory; a biological information memory operable to memorize the biological information; and a biological information storing unit operable to store the biological information measured by the measuring unit together with a measurement time of day into the biological information memory.

According to this construction, the dosage and biological information are respectively stored together with the calculation time of day and measurement time of day. Thus, these sets of information can be used for the health care.

More preferably, the dosage determination supporting apparatus may be further provided with: a physical condition measuring unit operable to measure a physical condition of the user; a physical condition memory operable to memorize a physical condition of the user; and a physical condition storing unit operable to store the physical condition of the user together with a measurement time of day into the physical condition memory.

According to this construction, the physical condition of the user can be stored into the memory. On account of this, these sets of information can be used for the health care. Here, as the physical condition of the user, the blood pressure, pulse, bodily temperature, etc. can be considered. Especially for a diabetic, it is important to measure the blood pressure because the patient may develop arteriosclerosis as a complication in some cases.

A syringe associated with another aspect of the present invention is a syringe which is capable of setting a dosage automatically, being provided with: a measuring unit operable to measure biological information obtained from one of inside or surface of a user's body; a dosage calculating unit operable to calculate a dosage on the basis of the biological information; and an injecting unit operable to inject the dosage of a medicine calculated by the dosage calculating unit.

According to this construction, the biological information is measured by the measuring unit, the dosage is calculated on the basis of the biological information, and the user is injected with the dosage of medicine. On this account, the physical condition of the user is perceived from the biological information and the dosage corresponding to it is precisely injected. In particular, this syringe combines a function for determining a dosage with a function for injecting medicine. For this reason, the user does not need to separately carry the dosage determination supporting apparatus and the syringe, and therefore the portability of the syringe improves.

A health-care supporting system associated with another aspect of the present invention is a heath-care supporting system which supports a health care of a user, being provided with: a dosage determination supporting apparatus operable to support a determination of a dosage to be administered to the user, with reference to a correspondence between biological information obtained from one of inside or surface of the user's body and the dosage of a medicine; and a server apparatus, which is connected to the dosage determination supporting apparatus via a network, operable to send the correspondence to the dosage determination supporting apparatus, wherein the dosage determination supporting apparatus includes: a measuring unit operable to measure the biological information of the user; a correspondence memory operable to memorize the correspondence; a calculating unit operable to calculate the dosage corresponding to the biological information, with reference to the correspondence; a notifying unit operable to notify the user of the dosage calculated by the calculating unit; and a correspondence rewriting unit operable to rewrite the correspondence that is received from the server apparatus and stored in the correspondence memory.

According to this construction, the biological information is measured by the measuring unit of the dosage determination supporting apparatus, the dosage is calculated on the basis of the correspondence between the biological information and the dosage, and the user is notified of it. On account of this, the physical condition of the user is perceived from the biological information, and the dosage corresponding to it is precisely determined. Also, because the latest correspondence is sent from the server apparatus, the dosage determination supporting apparatus can determine the optimal dosage for the user.

A server apparatus associated with another aspect of the present invention is a server apparatus which is connected, via a network, to a dosage determination supporting apparatus that supports a determination of a dosage of a medicine to be administered to a user, and which sends/receives various kinds of data to/from the dosage determination supporting apparatus, being provided with: a correspondence memory operable to memorize a correspondence between biological information obtained from one of inside and surface of the user's body and the dosage; a unit operable to receive the biological information from the dosage determination supporting apparatus; a calculating unit operable to calculate the dosage corresponding to the received biological information, with reference to the correspondence memory; and a unit operable to send the dosage calculated by the calculating unit to the dosage determination supporting apparatus.

According to this construction, even if the dosage determination supporting apparatus is unable to calculate the dosage, the dosage is calculated on the basis of the correspondence between the biological information and the dosage using the server apparatus and is sent to the dosage determination supporting apparatus. On this account, the physical condition of the user is perceived from the biological information, and the dosage corresponding to it is precisely determined.

A server apparatus associated with another aspect of the present invention is a server apparatus which is connected, via a network, to a dosage determination supporting apparatus that supports a determination of a dosage of a medicine to be administered to a user, and which sends/receives various kinds of data to/from the dosage determination supporting apparatus, being provided with: a correspondence memory operable to memorize a correspondence between biological information obtained from one of inside and surface of a user's body and the dosage; a unit operable to receive the biological information and the dosage from the dosage determination supporting apparatus; a judging unit operable to judge, with reference to the correspondence memory, whether the dosage corresponding to the received biological information can be authenticated; and a unit operable to send a judgment result given by the judging unit to the dosage determination supporting apparatus.

According to this construction, even when the user of the dosage determination supporting apparatus changes the dosage by correction etc., the user can inquire the server apparatus whether the dosage is valid. Owing to this, administration of an incorrect amount of medicine is prevented.

A method for historical-data communication associated with another aspect of the present invention is a method for historical-data communication according to a public key cryptosystem for a health-care supporting system which comprises a terminal apparatus used by a user and a server apparatus connected to the terminal apparatus and used by a health manager, being made up of: a step in which the terminal apparatus affixes a signature to the historical data regarding a health condition of the user, using a private key of the user; a step in which the terminal apparatus sends the historical data having the affixed signature to the server apparatus; a step in which the server apparatus receives the historical data having the affixed signature; and a step in which the server apparatus verifies the signature affixed to the historical data, using a registered public key of the user.

According to this construction, a signature is affixed to the historical data using the public key cryptosystem. Thus, the health manager can reliably know from which terminal apparatus of which user the historical data has been received, and the reliability of the historical data can be increased. Here, the health manager is a physician, a dentist, a pharmacist, a nurse, a hygienist, or a dietitian, for example.

A method for correspondence communication according to a public key cryptosystem for a health-care supporting system which comprises a dosage determination supporting apparatus used by a user and a server apparatus connected to the dosage determination supporting apparatus and used by a health manager, wherein the correspondence shows a correspondence between biological information obtained from one of inside or surface of the user's body and a dosage, and the dosage determination supporting apparatus obtains the dosage from the biological information, with reference to the correspondence, the method being provided with: a step in which the server apparatus affixes a signature to the correspondence using a private key of the health manager; a step in which the server apparatus sends the correspondence having the affixed signature to the dosage determination supporting apparatus; and a step in which the dosage determination supporting apparatus receives the correspondence having the affixed signature; and a step in which the dosage determination supporting apparatus verifies the signature affixed to the correspondence, using a registered public key of the health manager.

According to this construction, a signature is affixed to the correspondence using the public key cryptosystem. Thus, the user can reliably know from which server apparatus of which health manager the correspondence has been received, and the reliability of the correspondence in question can be increased.

### Brief Description of Drawings

FIG. 1 is an external view of a dosage determination supporting apparatus in a first embodiment of the present invention.
FIG. 2 is a diagram showing the hardware construction of a main body unit of the dosage determination supporting apparatus of the first embodiment of the present invention.
FIG. 3 is an example of an operation table, which is stored in an operation table storage area, showing the amounts of insulin corresponding to the blood sugar levels.
FIG. 4 is a flowchart showing a process flow of the dosage determination supporting apparatus.
FIG. 5 is a flowchart of historical data transmission processing performed by the dosage determination supporting apparatus that a user uses.
FIG. 6 is a flowchart of historical data reception processing performed by a PC (Personal Computer) that a physician uses.
FIG. 7 is an external view of a dosage determination supporting apparatus in a second embodiment of the present invention.
FIG. 8 is a diagram showing the hardware construction of a main body unit of the dosage determination supporting apparatus of the second embodiment.
FIG. 9 is an external view of a syringe in a third embodiment of the present invention.
FIG. 10 is a diagram showing the hardware construction of a main body unit of the syringe.
FIG. 11 is a flowchart showing a process flow of the syringe.
FIG. 12 is an external view of a syringe in a fourth embodiment of the present invention.
FIG. 13 is a diagram showing the hardware construction of a main body unit of the syringe.
FIG. 14 is a flowchart showing a process flow of the syringe.
FIG. 15 is a rough schematic diagram showing a main part of an adjusting unit.
FIG. 16 is a block diagram showing the construction of a health-care supporting system of a fifth embodiment of the present invention.
FIG. 17 is a flowchart of a process flow of the dosage determination supporting apparatus.
FIG. 18 is a flowchart of arithmetic processing for the amount of insulin performed by a PC.
FIG. 19 is a flowchart of authentication processing for the amount of insulin performed by the PC.

### Best Mode for Carrying Out the Invention

### (First Embodiment)

The following is an explanation of a dosage determination supporting apparatus of the first embodiment of the present invention, with reference to the drawings. Note that, in the embodiments of the present invention described below, the same numeral will be accordingly given to the components that have the same function. On this account, the detailed explanation for them is accordingly omitted.

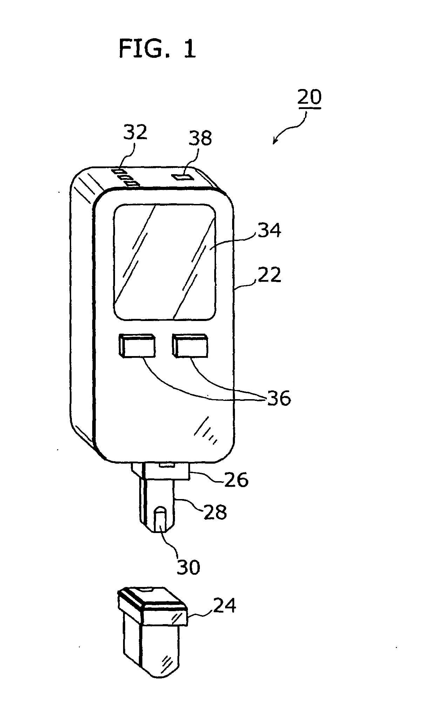

FIG. 1 is an external view of a dosage determination supporting apparatus of the first embodiment of the present invention. A dosage determination supporting apparatus 20 is composed of a main body unit 22 and a sensor case 24.

The main body unit 22 includes a sensor 28, a sensor attachment unit 26, a displaying unit 34, a data inputting unit 36, an input/output terminal 38, a speaker 32, and a clock function (not shown).

The sensor attachment unit 26 is located at the lower part of the main body unit 22 and can equip with the sensor 28. The sensor 28 is located at the end and includes: an applying point unit 30 to which sample solution (for example, blood) is applied; a reagent layer (not shown) containing the enzymes (for example, glucose oxidase) that react to the glucose in the sample solution; and a pair of electrodes (not shown). The reagent layer further contains electron transporters (for example, potassium ferricyanide) preferably.

The sensor case 24 has a fit unit between the sensor attachment unit 26 and itself, so as to prevent dust and the like from entering into the main body unit 22 by being attached to the sensor attachment unit 26 at the time of carrying along and so forth. Moreover, if the sensor case 24 is attached and carried in the state where the sensor 28 has been attached, the measurement of blood sugar level can be quickly prepared while away from home. In this case, the moisture absorption of the sensor 28 may be prevented by having the sensor case 24 contain an absorbent.

On the displaying unit 34, information such as the measured blood sugar level and the amount of insulin to be injected is displayed. These sets of information can be also notified by voice through the speaker 32 for the patient whose vision has deteriorated.

The data inputting unit 36 is used for inputting information into the main body unit 22.

The input/output terminal 38 is provided for inputting/outputting data into/from outside resources.

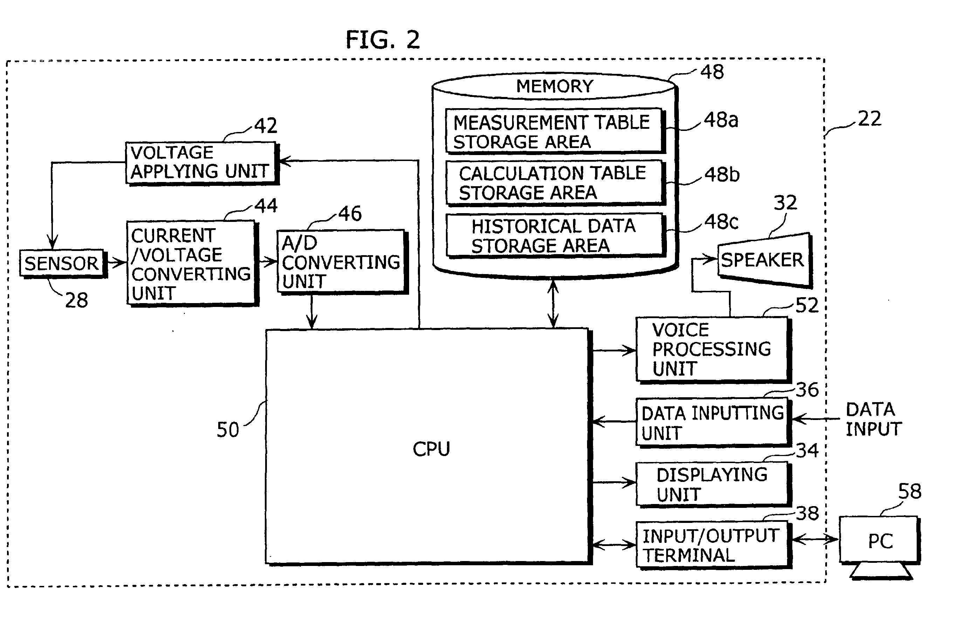

FIG. 2 is a diagram showing the hardware construction of the main body unit 22 of the dosage determination supporting apparatus 20. The main body unit 22 includes a voltage applying unit 42, a current/voltage converting unit 44, an A/D (Analog to Digital) unit 46, a memory 48, a CPU (Central Processing Unit) 50, a voice processing unit 52, the data inputting unit 36, and the displaying unit 34.

The voltage applying unit 42 applies voltage to the sensor 28 and plays a role as a drive power source for the sensor 28.

The current/voltage converting unit 44 converts the current outputted from the electrodes of the sensor 28 into the voltage and then outputs it.

The A/D converting unit 46 converts the voltage value outputted from the current/voltage converting unit 44 into a pulse.

The CPU 50 performs arithmetic processing etc. to calculate the blood sugar level from the pulse value outputted from the A/D converting unit 46.

The memory 48 includes: a measurement table storage area 48a for storing a measurement table showing the correspondence between the pulse value outputted from the current/voltage converting unit 44 and the blood sugar level; an operation table storage area 48b for storing an operation table showing the correspondence between the blood sugar level and the amount of insulin; and a historical data storage area 48c for storing historical data, such as the measured blood sugar levels, the calculated amounts of insulin, and dates/times.

The voice processing unit 52 performs voice processing in response to a direction from the CPU 50 and outputs the voice through the speaker 32.

The main body unit 22 is connected to an external PC (Personal Computer) 58 via the input/output terminal 38.

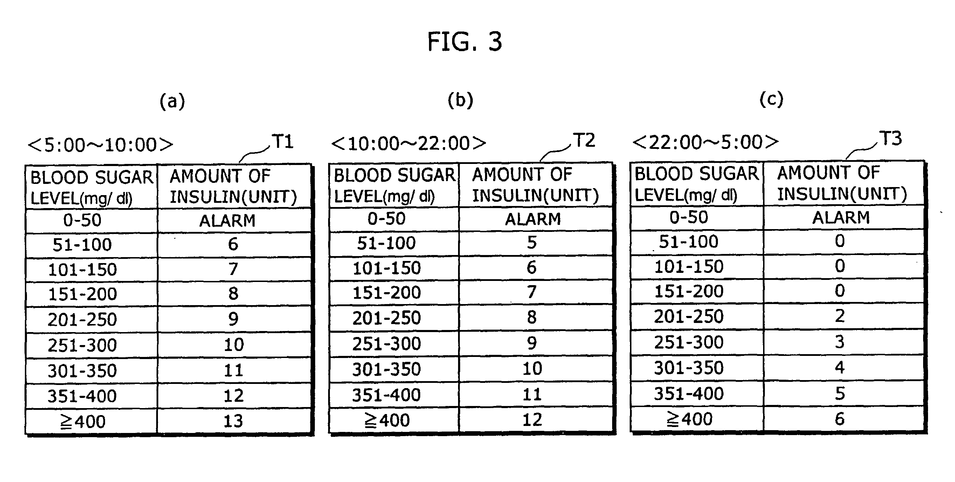

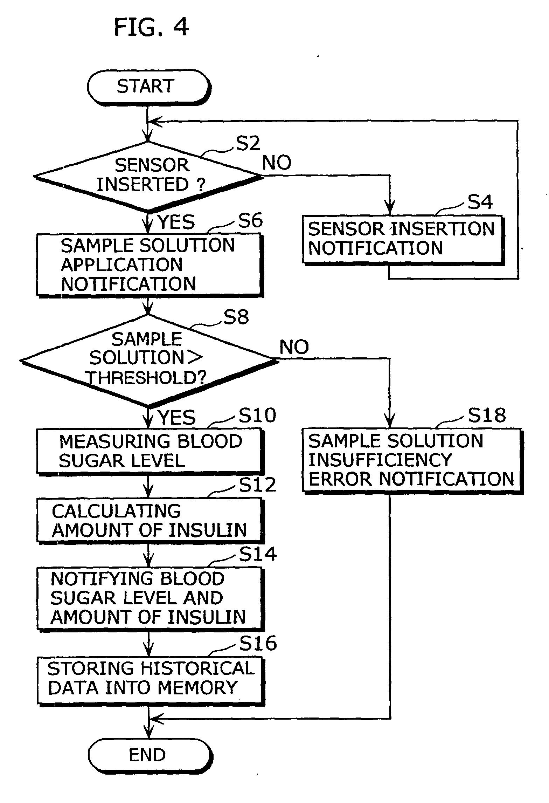

Hereafter, the operations of the user and the dosage determination supporting apparatus 20 are explained with reference to FIGS. 3 and 4. Note that although the explanation will be given for the measurement using bodily fluid of a human body, particularly blood, as the sample solution, matrix fluid etc. may be used as the bodily fluid.

In advance of the measurement of blood sugar level, the user has the operation table storage area 48b of the memory 48 store the operation table. As a method for having the operation table stored, there are a method by inputting data of the operation table from the data inputting unit 36 and a method by connecting the PC 58 to the input/output terminal 38 and inputting the data of the operation table from the PC 58. Note that the operation table is formed under the direction of a physician in accordance with various conditions, such as a patient's individual difference, injection time of day, and an insulin type.

FIG. 3 is an example of the operation table, which is stored in the operation table storage area 48b, showing the amounts of insulin corresponding to the blood sugar levels. In FIG. 3, the amount of insulin (unit) corresponding to the blood sugar level (mg/dl) shows a unit in a case where fast acting insulin is used. Here, the "unit" indicates the strength of biological activity of insulin, and one unit is 0.0353mg when converting it into a weight according to an international standard article.

FIG. 3 (a) shows an operation table T1 (assuming that an insulin injection is given before breakfast) of 5:00≤t<10:00 when the time of day to measure the blood sugar level is set to t. FIG. 3 (b) shows an operation table T2 (assuming that an insulin injection is given before lunch or dinner) of 10:00≤t<21:00. FIG. 3 (c) shows an operation table T3 (assuming that an insulin injection is given before a between-meal snack at night) of 21:00≤t<5:00. For example, in the operation table T1, when the blood sugar level is 101 to 150 (mg/dl), the amount of insulin should be 7 (units).

FIG. 4 is a flowchart showing a process flow of the dosage determination supporting apparatus 20. As a preparation to measure the blood sugar level, the user removes the sensor case 24 and fits the sensor 28 to the sensor attachment unit 26.

When the user turns ON the power switch (not shown) of the dosage determination supporting apparatus 20, the processing of the dosage determination supporting apparatus 20 is started. Note that the power switch of the dosage determination supporting apparatus 20 may be automatically turned on when the sensor 28 is inserted into the sensor attachment unit 26.

The CPU 50 judges whether the sensor 28 is securely fitted to the sensor attachment unit 26 (S2). More specifically, the CPU 50 judges whether the sensor 28 is securely fitted by detecting conduction of the switch (not shown) which is brought into conduction when the sensor 28 is fitted.

If the conduction of the sensor 28 is not verified within a set period of time (NO in S2), it is judged that the sensor 28 has not been inserted, so that a sensor insertion notification is provided (S4). To be more specific, the displaying unit 34 is directed to display so as to call attention to the insertion of the sensor 28. Moreover, an alarm sound or message on which the voice processing has been performed by the voice processing unit 52 is heard through the speaker 32. Especially for the patient whose vision has deteriorated due to diabetic complication, the notification by voice is preferable. Therefore, when the "notification" is referred to in the present specification, it indicates the notification by the screen display or the notification by voice. When the user inserts the sensor 28, the processing will be started again from S2.

If the conduction of the sensor 28 is verified within the set period of time (YES in S2), the CPU 50 provides a notification so that the user will apply the blood to the applying point unit 30 of the sensor 28 (S6). On the basis of this notification, the user extracts the blood as sample solution from his/her own body and applies this extracted blood to the applying point unit 30 of the sensor 28.

Next, the CPU 50 judges whether the amount of the applied blood is sufficient for measuring the blood sugar level (S8). To be more specific, the voltage is previously placed between the electrodes of the sensor 28 from the voltage applying unit 42. The CPU 50 detects the application of the blood by detecting a change in the pulse value outputted from the A/D converting unit 46 via the current/voltage converting unit 44, and then judges whether the sufficient amount of blood has been applied on the basis of the variations in the pulse value after a set period of time.

When the amount of blood is equal to or below a threshold (the amount sufficient for the measurement) (NO in S8), the amount of the sample solution is judged to be insufficient, so that a sample solution insufficiency error notification is provided (S18), where the measurement processing is terminated. Accordingly, an incorrect measurement of the blood sugar level by the dosage determination supporting apparatus 20 can be prevented.

When the amount of blood is larger than the threshold (YES in S8), the CPU 50 measures the blood sugar level (S10). More specifically, when the applied blood is sucked inside the sensor 28, the reagent layer of the sensor 28 dissolves. Enzyme reaction proceeds between enzyme contained in the reagent layer and glucose in the blood, and at the same time an electron transporter (such as ferrocyanide ion) of a reduced form is produced. The CPU 50 has the voltage applying unit 42 apply the voltage between the electrodes of the sensor 28. At this time, the electron transporter of the reduced form, i.e., the current according to the amount of glucose in the blood, is passing between the electrodes of the sensor 28. The current/voltage converting 44 converts the current passing between the electrodes into voltage and then outputs it. The A/D converting unit 46 converts the voltage outputted from the current/voltage converting unit 44 into a pulse and then inputs it into the CPU 50. The CPU 50 calculates the blood sugar level with reference to the measurement table stored in the measurement table storage area 48a of the memory 48, the table showing the correspondence between the pulse value outputted from the current/voltage converting unit 44 and the blood sugar level.

After the measurement of the blood sugar level, the CPU 50 calculates the amount of insulin to be administered (S12). To be more specific, the CPU 50 calculates the amount of insulin from the value of the measured blood sugar level with reference to the operation tables stored in the operation table storage area 48b of the memory 48. As one example, an explanation is given for a case where the operation tables stored in the operation table storage area 48b are the operation tables T1 to T3 shown in FIG. 3. The CPU 50 detects the measurement time of day of the blood sugar level using a clock function provided for the main body unit 22 and, according to the measurement time, selects one of the operation tables T1 to T3. With reference to the selected operation table, the CPU 50 calculates the amount of insulin corresponding to the measured blood sugar level. This allows the user to save the time and effort to calculate the amount of insulin by himself/herself, and also allows the time taken from the measurement of the blood sugar level to the insulin injection to be reduced. In addition, the correct amount of insulin to be injected can be calculated.

The CPU 50 notifies the user of the measured blood sugar level and the calculated amount of insulin (S14). Also, the CPU 50 stores the measured blood sugar level, the calculated amount of insulin, and the measurement time and date into the historical data storage area 48c of the memory 48 (S16).

By the processing as explained above, the user can know the amount of insulin to be injected from the measured blood sugar level. On the basis of this amount of insulin, the user injects with insulin.

Moreover, the user can send the historical data stored in the above-mentioned historical data storage area 48c to the PC 58 of the physician via the network, and can receive an appropriate treatment from the physician. Based on the historical data, the physician can make a change to the operation table stored in the operation table storage area 48b of the memory 48 of the dosage determination supporting apparatus 20 that the user uses.

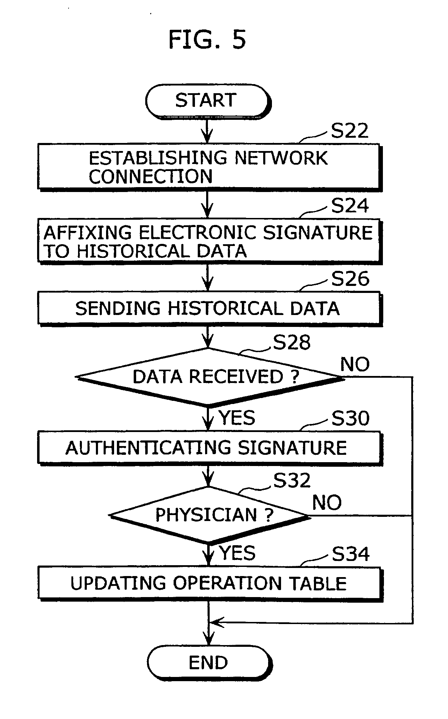

FIG. 5 is a flowchart of historical data transmission processing performed by the dosage determination supporting apparatus 20 that the user uses. It should be noted that RSA (Rivest Shamir Adleman) algorithm that is a kind of the public key cryptosystem is used for sending/receiving data between the dosage determination supporting apparatus 20 that the user uses and the PC 58 that the physician uses. For this reason, a public key and a private key of the user and a public key of the physician are previously stored in the memory 48 of the dosage determination supporting apparatus 20. Moreover, note that the public key of the user who is a patient and the public key and private key of the physician are previously stored in the PC 58 that the physician uses.

The CPU 50 of the dosage determination supporting apparatus 20 establishes connection with a network such as the Internet via the input/output terminal 38 (S22). The CPU 50 affixes an electronic signature to the historical data using the private key of the user (S24) and sends the historical data with the electronic signature to the PC 58 of the physician (S26). The CPU 50 waits to receive the data from the PC 58 of the physician. When the data cannot be received even after a predetermined period of time (NO in S28), the processing is terminated. When the data is received within the predetermined period of time (YES in S28), the signature affixed to the data is verified using the public key of the physician (S30). If the received data is not the one sent from the physician (NO in S32), the processing is terminated. If the received data is the one sent from the physician (YES in S32), the CPU 50 overwrites the operation table storage area 48b with the operation table included in the data to update the operation table (534).

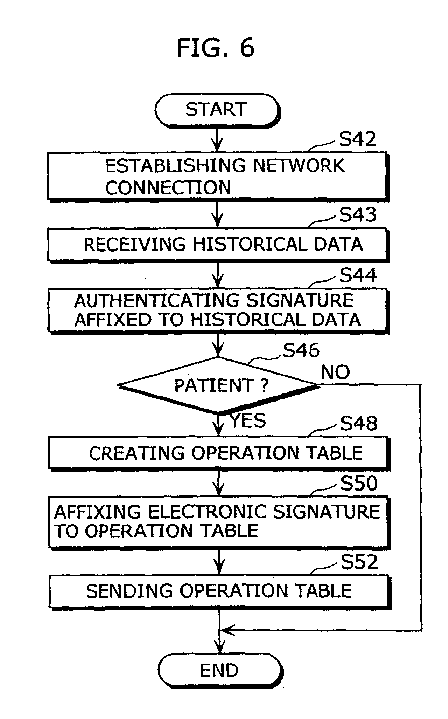

FIG. 6 is a flowchart of historical data reception processing performed by the PC 58 that the physician uses. In response to the network connection establishment processing by the dosage determination supporting apparatus 20 that is shown in FIG. 5, the PC 58 establishes connection with the network (S42). The PC 58 receives the historical data from the dosage determination supporting apparatus 20 (S43). The public keys of patients are stored in the PC 58 corresponding to the number of patients that the physician takes charge of. For this reason, the PC 58 verifies the electronic signature affixed to the historical data using the public key of the patient so as to check which patient's historical data it is (S44). If the signature affixed to the historical data is perceived not to be any of the patients' (NO in S46), the processing is terminated.

If the signature affixed to the historical data is one of the patients' (YES in S46), the PC 58 re-creates an operation table based on the historical data of the patient in question (S48). It should be noted that when the operation table is created, the physician participates in the data creation. When the operation table is created, the PC 58 affixes an electronic signature to the created operation table using the private key of the physician (S50), and sends the operation table to the dosage determination supporting apparatus 20 (S52).

As explained above, according to the present embodiment, the dosage determination supporting apparatus 20 calculates the amount of insulin to be administered to the user from the measurement result of the measured blood sugar level, by reference to the operation table stored in the operation table storage area 48b. Owing to this, the user can accurately know the amount of insulin to be administered.

Moreover, the historical data including the measurement result of the blood sugar level etc. is sent to the PC 58 of the physician, and based on the historical data, the operation table is re-created again. Accordingly, the dosage can be accurately determined in keeping with physical condition of the user.

Furthermore, at the time of the communication between the dosage determination supporting apparatus 20 and the PC 58, the signature is affixed to the data to be sent/received according to the public key cryptosystem so that the source of data is identified. For this reason, if viewed from the user's side, it is reliably guaranteed that the operation table to be updated is created by the physician who takes charge of the user. On account of this, the dosage determination supporting apparatus 20 can be used with a sense of security. In addition, if viewed from the physician's side, it is reliably guaranteed that the received historical data belongs to his/her patient. Accordingly, the operation table in keeping with physical condition of the user can be created without fail.

### (Second Embodiment)

The following is an explanation of a dosage determination supporting apparatus in the second embodiment of the present invention, with reference to the drawings.

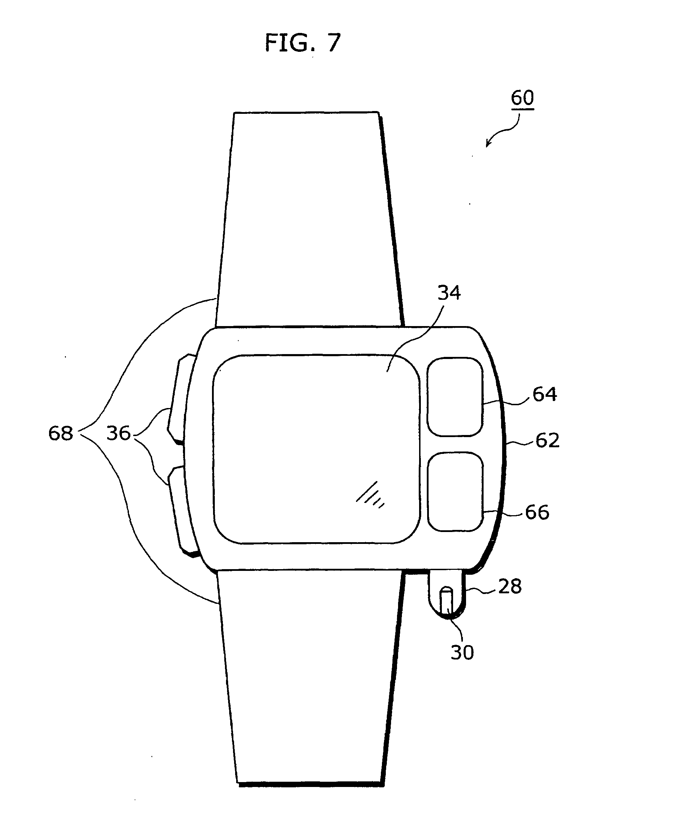

FIG. 7 is an external view of the dosage determination supporting apparatus in the second embodiment of the present invention. A dosage determination supporting apparatus 60 is a wristwatch-type dosage determination supporting apparatus 60, and is provided with a main body unit 62, and a belt 68.

The main body unit 62 includes a sensor 28, a displaying unit 34, a data inputting unit 36, a light sensor 64, a pulse sensor 66, a speaker (not shown), an infrared communication unit (not shown), and a clock function (not shown).

The sensor 28 is located at the lower part of the main body unit 62, and is attached to the main body unit 62 only when it is used.

The light sensor 64 is used for biometrics authentication. The biometrics authentication is an authentication method of identifying an individual by a fingerprint, the pattern of blood vessel, etc. The pulse sensor 66 is used for measuring a pulse. Note that it is possible to measure the blood pressure based on the pulse.

The infrared communication unit is connected to an external PC 58 according to the short-distance wireless communication technology typified by Bluetooth.

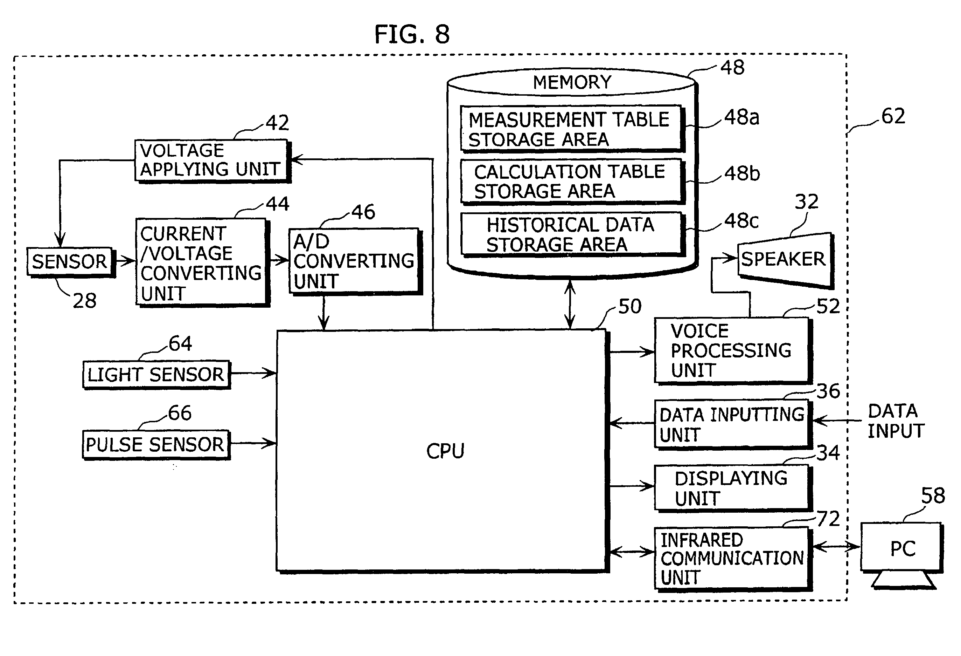

FIG. 8 is a diagram showing the hardware construction of the main body unit 62 of the dosage determination supporting apparatus 60. The main body unit 62 is provided with an infrared communication unit 72 in place of the input/output terminal 38 in the main body unit 22 of the dosage determination supporting apparatus 20 shown in FIG. 2, and is also newly provided with the light sensor 64 and the pulse sensor 66.

As described above, the dosage determination supporting apparatus 60 can measure the blood sugar level using the sensor 28. The method of measuring the blood sugar level using the sensor 28 is the same as the method of measuring the blood sugar level that is shown in FIG. 4 in the first embodiment. It should be noted that the pulse and blood pressure measured using the pulse sensor 66 are stored together with the measurement time into the historical data storage area 48c of the memory 48.

The sending/receiving processing for the historical data via the infrared communication unit 72 is the same as that shown in FIGS. 5 and 6.

Moreover, it is possible to input data, such as an operation table, from the data inputting unit 36. In this case, it is necessary to previously authenticate a person who inputs the data. In order to do this, characteristic data extracted through the image processing from the image data of a fingerprint of the physician or patient is previously stored in the memory 48.

When inputting the data from the data inputting unit 36, the user first brings his/her finger in contact with a light-receptive part of the light sensor 64. The CPU 50 reads the image data of the fingerprint via the light sensor 64 and extracts the characteristic data by performing the image processing on the image data. The CPU 50 judges whether the extracted characteristic data matches with the characteristic data stored in the memory 48. Only when these sets of the characteristic data are judged to match with each other, the CPU 50 receives inputs from the data inputting unit 36.

As explained above, according to the present embodiment, historical information such as a pulse and blood pressure can be sent to the physician, in addition to the operation and effect of the first embodiment. On account of this, the physician can know the physical condition of the patient in more detail and offer an operation table more suitable for the patient. Especially, a diabetic tends to cause arteriosclerosis as complication. For this reason, the blood pressure of a diabetic is particularly important to know the physical condition as well.

### (Third Embodiment)

The following is an explanation about a syringe of the third embodiment of the present invention, with reference to the drawings.

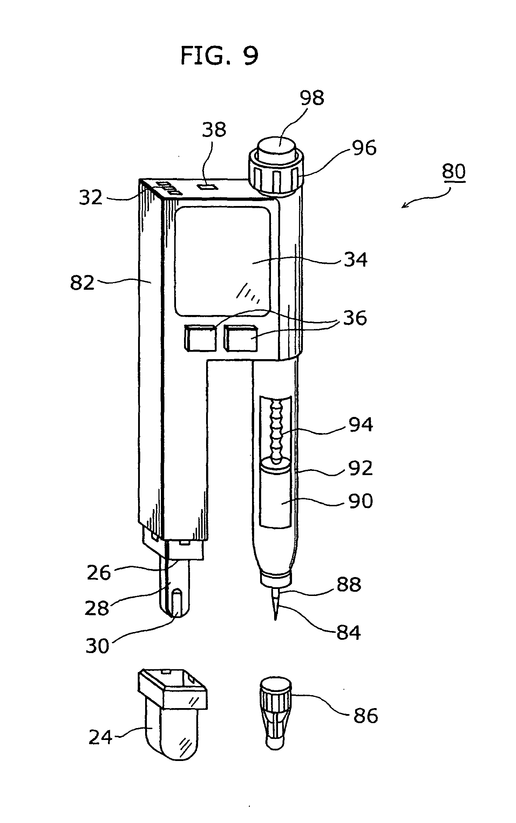

FIG. 9 is an external view of the syringe in the third embodiment of the present invention. A syringe 80 is provided with a main body unit 82, a sensor case 24, and a needle sheath 86.

The main body unit 82 includes a sensor 28, a sensor attachment unit 26, a displaying unit 34, a data inputting unit 36, an input/output terminal 38, a speaker 32, an injection needle 84, a needle attachment unit 88, an insulin cartridge 90, an insulin holder 92, a piston 94, a setting unit 96, an injection button 98, and a clock function (not shown).

The needle attachment unit 88 is located at the tip of the main body unit 82 and can equip with the injection needle 84. Here, it is preferable that the sensor 28 and the injection needle 84 which are to be attached to the main body unit 82 project from the main body unit 82 in the same direction. Thereby, the user can localize attention in one place, that is to be brought to the sensor 28 and the injection needle 84 to which bodily fluid such as blood is likely to adhere.

Each of the sensor case 24 and the needle sheath 86 has a fit unit between the sensor attachment unit 26 and itself, and they are attached to the main body unit 82 when the main body unit 82 is not being used, like at the time of carrying along. Owing to this, dust and the like is prevented from entering into the main body unit 22 at the time of carrying along. Moreover, if the sensor case 24 is attached and carried in the state where the sensor 28 has been attached, the measurement of blood sugar level can be quickly prepared while away from home. In this case, the moisture absorption of the sensor 28 may be prevented by having the sensor case 24 contain an absorbent.

In the main body unit 82, a device for measuring the blood sugar level and a device for injecting insulin are provided as one piece in one enclosure. Insulin is contained in the insulin cartridge 90. The insulin holder 92 is a cylinder in shape and holds the insulin cartridge 90 inside. The piston 94 discharges the insulin in the insulin cartridge 90 into the injection needle 84 through the needle attachment unit 88. The user uses the setting unit 96 in order to set the injection amount of insulin to be discharged. The user uses the injection button 98 in order to inject insulin.

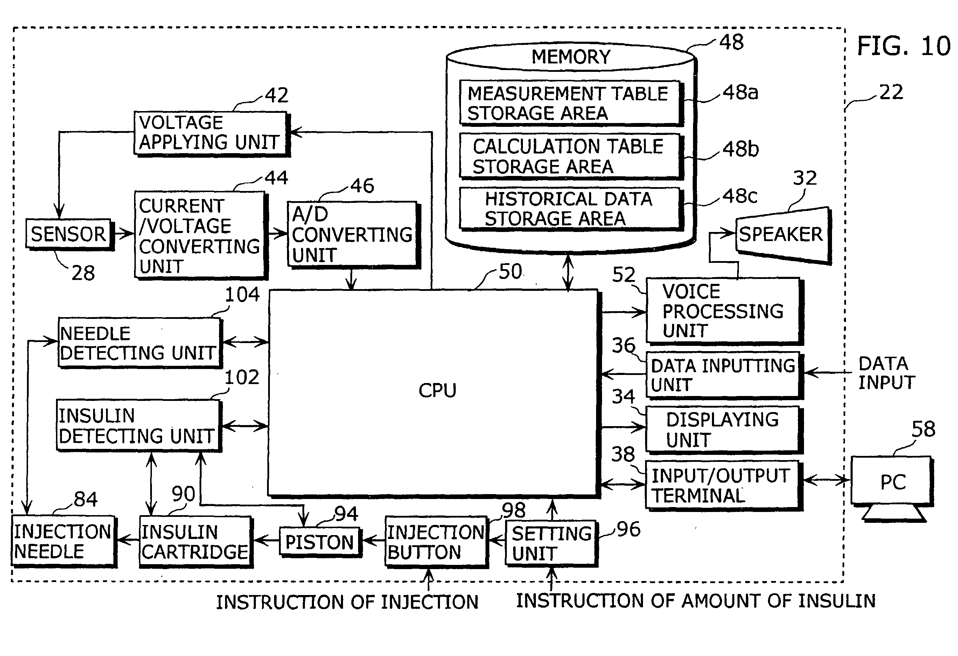

FIG. 10 is a diagram showing the hardware construction of the main body unit 82 of the syringe 80. In addition to the construction of the main body unit 22 of the dosage determination supporting apparatus 20 shown in FIG. 2, the main body unit 82 is newly provided with the injection needle 84, the insulin cartridge 90, the piston 94, the injection button 98, the setting unit 96, an insulin detecting unit 102, and a needle detecting unit 104.

In the historical data storage area 48c, the measured blood sugar level, the calculated amount of insulin, and the set amount of insulin are stored together with the measurement time of day, the calculation time of day, and the set time of day.

The insulin detectingunit 102 detects information regarding insulin, such as the remaining amount of insulin and an insulin type. The needle detecting unit 104 detects whether the injection needle 84 is attached or not.

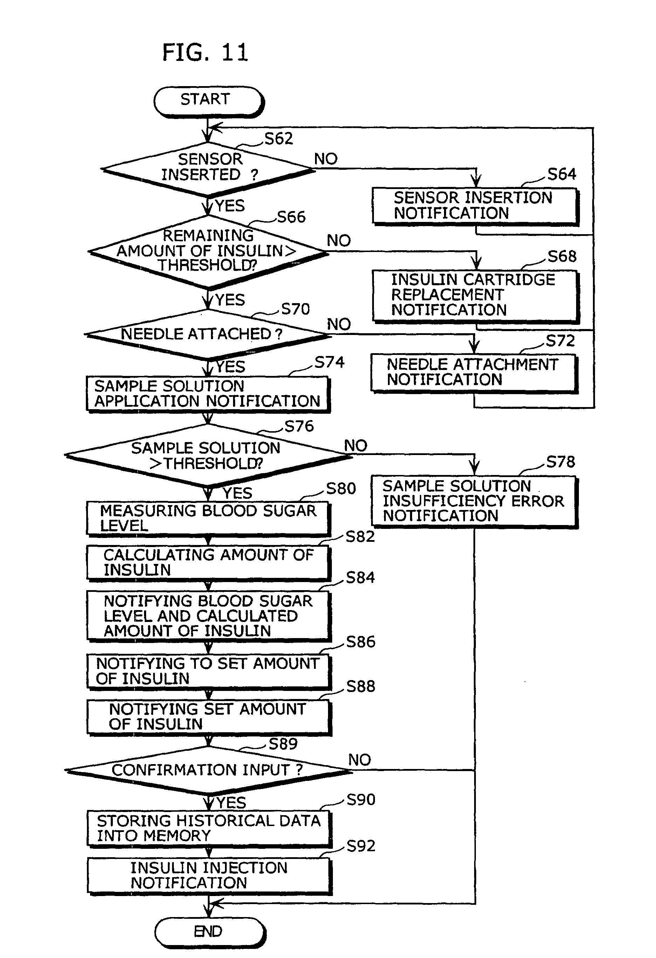

FIG. 11 is a flowchart showing a process flow of the syringe 80. As a preparation for the measurement of blood sugar level and insulin injection, the user removes the sensor case 24 and the needle sheath 86 and attaches the sensor 28 and injection needle 84 respectively to the sensor attachment unit 26 and the needle attachment unit 88. After this, it is preferable to reattach the needle sheath 86 so that the injection needle 84 will not accidentally stick in the user's hand during the measurement of blood sugar level. Moreover, the user checks if the sufficient amount of insulin is contained in the insulin cartridge 90 and sets a new insulin cartridge 90 as necessary.

The processing by the syringe 80 is stared by the user turning ON a power switch (not shown) of the main body unit 82. The CPU 50 judges whether the sensor 28 is securely attached to the sensor attachment unit 26 (S62). This judgment processing is the same as the sensor insertion judgment processing (S2) in the first embodiment shown in FIG. 4.

If the conduction of the sensor 28 is not verified within a set period of time (NO in S62), it is judged that the sensor 28 has not been inserted, so that a sensor insertion notification is provided (S64). This notification processing is the same as the sensor insertion notification processing (S4) in the first embodiment show in FIG. 4.

If the conduction of the sensor 28 is verified within the set period of time (YES in S2), the CPU 50 judges whether the remaining amount of insulin is larger than a predetermined threshold (the amount of insulin required for one injection) (S66). More specifically, the insulin detecting unit 102 shown in FIG. 10 verifies whether the insulin cartridge 90 is attached to the insulin holder 92, and detects the remaining amount of insulin from the position of the piston 94.

If the insulin cartridge 90 is not attached or the remaining amount of insulin is equal to or below the predetermined threshold (NO in S66), the CPU 50 recognizes that there is no remaining amount of insulin and provides a notification for replacement of the insulin cartridge 90 (S68). On account of this, it is possible to avoid a case where the remaining amount of insulin is running short and the set amount of insulin cannot be injected. By the user attaching a new insulin cartridge 90 to the insulin holder 92, the sensor insertion judgment processing (S62) is performed again. It should be noted that the insulin detecting unit 102 may be provided with a function for detecting an insulin type from information regarding the insulin cartridge 90.

If the insulin cartridge 90 is attached and the remaining amount of insulin is larger than the threshold (YES in S62 and YES in S66), the CPU 50 judges whether the injection needle 84 is securely attached to the needle attachment unit 88 (S70). To be more specific, the needle detecting unit 104 detects the conduction state of a switch (not shown) that conducts when the set injection needle 84 is attached to the needle attachment unit 88. On the basis of this conduction state, whether the injection needle 84 is securely attached or not is detected.

When the needle detecting unit 104 cannot verify the conduction of the switch (NO in S70), the CPU 50 recognizes that the injection needle 84 is not securely attached and provides a needle attachment notification (S72). Owing to this, a risk that the injection needle 84 is detached from the needle attachment unit 88 during the measurement of blood sugar level or during the insulin injection can be avoided. When the user attaches the injection needle 84 to the needle attachment unit 88, the sensor insertion judgment processing (S62) is performed again.

When the conduction of the switch is verified by the needle detecting unit 104 and the injection needle 84 is judged to be securely attached to the needle attachment unit 88 (YES in S70), the CPU 50 provides a notification so that the user will apply the blood to the applying point unit 30 of the sensor 28 (S74). Based on this notification, the user extracts the blood as a sample solution from his/her own body and applies the extracted blood to the applying point unit 30 of the sensor 28.

Next, the CPU 50 judges whether the amount of the applied blood is sufficient for measuring the blood sugar level (S76). This judgment processing is the same as the sample solution judgment processing (S8) shown in FIG. 4 in the first embodiment.

When the amount of blood is equal to or below a threshold (the amount sufficient for the measurement) (NO in S76), the amount of the sample solution is judged to be insufficient, so that a sample solution insufficiency error notification is provided (S78), where the processing is terminated. Accordingly, an incorrect measurement of the blood sugar level by the dosage determination supporting apparatus 20 and an incorrect judgment of the amount of insulin incident to it can be prevented.

When the amount of blood is larger than the threshold (YES in S76), the CPU 50 measures the blood sugar level (S80). This blood sugar level measurement processing is the same as the blood sugar level measurement processing (S10) explained with reference to FIG. 4 in the first embodiment.

After the end of the blood sugar level measurement, the CPU 50 calculates the amount of insulin to be administered (S82). This insulin amount calculation processing is the same as the insulin amount calculation processing (S12) explained with reference to FIG. 4 in the first embodiment.

The CPU 50 notifies the user of the measured blood sugar level and the calculated amount of insulin (S84). Moreover, the CPU 50 provides a notification so that the user will set the amount of insulin to be injected (S86). By reference to the notified blood sugar level and amount of insulin, the user sets the amount of insulin to be injected by manually adjusting the setting unit 96, taking consideration of the amounts of diet, exercise, etc. if necessary. At this time, the CPU 50 provides the set amount of insulin together with a confirmation message asking whether or not to consent to inject that amount of insulin (S88). For a set of period of time, the CPU 50 waits for the user to input a confirmation of consenting to inject the insulin. If there is no such confirmation input (No in S89), the CPU 50 terminates the processing.

If there is such confirmation input (YES in S89), the CPU 50 stores the measured blood sugar level, the calculated amount of insulin, and the set amount of insulin together with at least the date or time of the measurement into the historical data storage area 48c (S90).

Finally, the CPU 50 notifies the user to inject the insulin (S92). In response to this notification, the user inserts the injection needle 84 into his/her arm etc. and then pushes the injection button 98. In consequence of this, the piston 94 discharges the insulin inside the insulin cartridge 90 into the injection needle 84. The discharged insulin is injected into the user's body through the injection needle 84.

Moreover, the sending/receiving processing for the historical data via the input/output terminal 38 is the same as that shown in FIGS. 5 and 6.

Moreover, it is possible to input data, such as an operation table, from the data inputting unit 36. In this case, note that account names and passwords previously assigned to the physician and user are stored in the memory 48. When data, such as an operation table, is to be inputted from the data inputting unit 36 or when the historical data is to be referred to, inputs of the account name and password are requested prior to these processes. Following this request, the physician or user inputs the account name and password, and if they match with those stored in the memory 48, the above processes are permitted.

As explained up to this point, the present embodiment has operations and effects as described below in addition to the operations and effects in the above-mentioned embodiments.

That is, the syringe 80 combines a function for measuring the blood sugar level and a function for injecting insulin. For this reason, the user does not need to separately carry a blood sugar level measuring device and an insulin syringe, so that the portability of the syringe improves.

Moreover, the user can measure the blood sugar level, holding the syringe 80 with one hand, and can inject insulin. In this way, there is no need for a changing of devices between the measurement of the blood sugar level and the insulin injection. Moreover, the syringe 80 provides notifications so as to call the user's attention to apply the sample solution, to adjust the set amount of insulin, and to inject it. The user can thereby know the procedures and timings of the measurement of blood sugar level and the injection of insulin. Accordingly, the operability of the syringe improves.

Furthermore, the syringe 80 stores the measured blood sugar level, the calculated amount of insulin, and the set amount of insulin (the amount of injected insulin) together with at least the date or time of the measurement into the memory 48. By means of this, the user can use this history for a grasp of his/her medical condition and self-supervision. This is because it is important to grasp where the responsible insulin of the blood sugar level is, i.e., to grasp that the injected insulin is responsible for the blood sugar level of when. In particular, when hypoglycemia occurs, it is often caused by the incorrect amount of injected insulin. Therefore, it is a big advantage for the user to check the history, such as the blood sugar level and the amount of injected insulin, later on.

### (Fourth Embodiment)

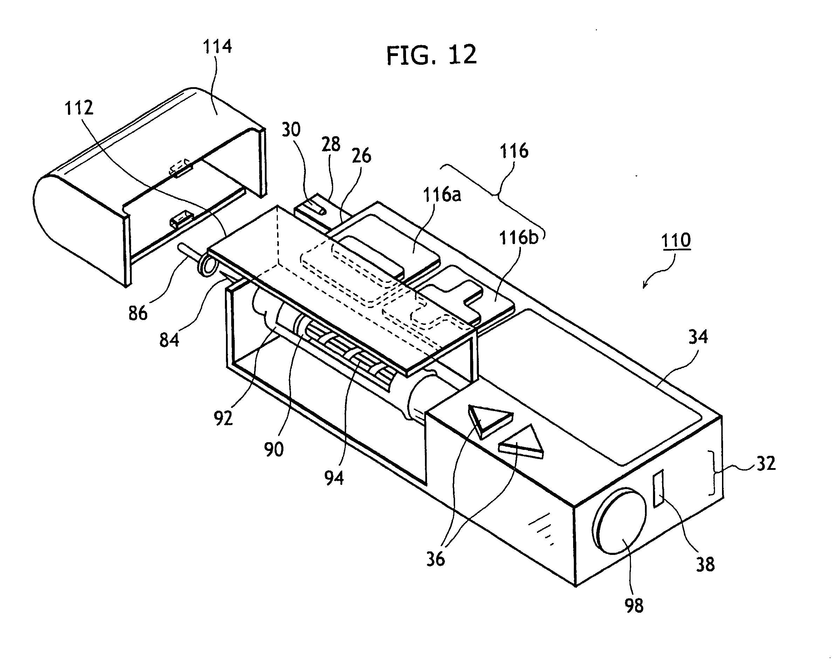

The following is an explanation about a syringe of the fourth embodiment of the present invention, with reference to the drawings. FIG. 12 is an external view of the syringe of the fourth embodiment of the present invention. A syringe 110 is provided with a main body unit 112 and a housing case 114.

The main body unit 112 includes a sensor 28, a sensor attachment unit 26, a displaying unit 34, a data inputting unit 36, an input/output terminal 38, a speaker 32, an injection needle 84, a needle attachment unit 88, an insulin cartridge 90, an insulin holder 92, a piston 94, an injection button 98, a correction amount inputting unit 116, and a clock function (not shown).

The correction amount inputting unit 116 is provided in order to correct the set amount of insulin by increasing/decreasing through inputs, and is composed of a correction buttons 116a and 116b. The user can decrease the set amount of insulin by pushing the correction button 116a that has a projection and depression forming the shape of a minus sign on its surface. Also, the set amount of insulin can be increased by pushing the correction button 116b that has a projection and depression forming the shape of a plus sign on its surface.

The housing case 114 can house the sensor 28 and the injection needle 84. When the syringe 110 is not being used, like at the time of carrying along, the housing case 114 is attached to the main body unit 112. After the end of an insulin injection, the user can attach the housing case 114 to the main body unit 112, with the sensor 28 and the injection needle 84 still being attached.

Since it is difficult to dispose of the sensor 28 or injection needle 84 to which bodily fluid has been adhered while away from home and so forth, the convenience for the user improves. Moreover, a cleanup after the injection can be quickly done. Furthermore, when it is carried with the housing case being attached in a state where the sensor 28 and the injection needle 84 have been attached, a preparation for the blood sugar level measurement and the insulin injection can be quickly made while away from home and so forth. In this case, the housing case 114 may include an absorbent. By means of this, the moisture absorption of the sensor 28 can be prevented.

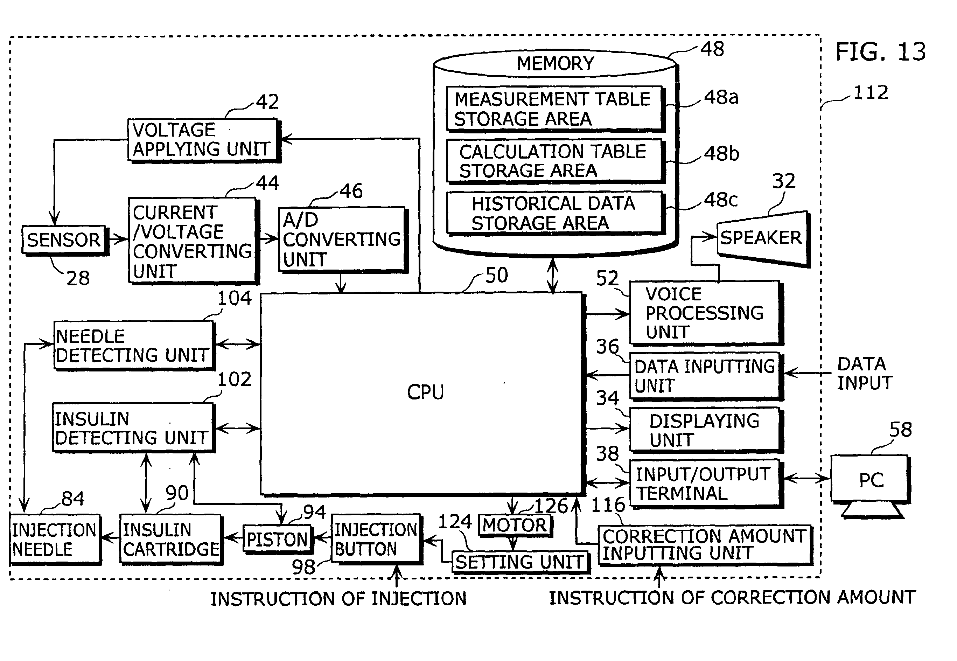

FIG. 13 is a diagram showing the hardware construction of the main body unit 112 of the syringe 110. The main body unit 112 is provided with a setting unit 124 replacing the setting unit 96 in the construction of the main body unit 82 of the syringe 80 shown in FIG. 10, and is newly provided with a motor 126 for driving the setting unit 124.

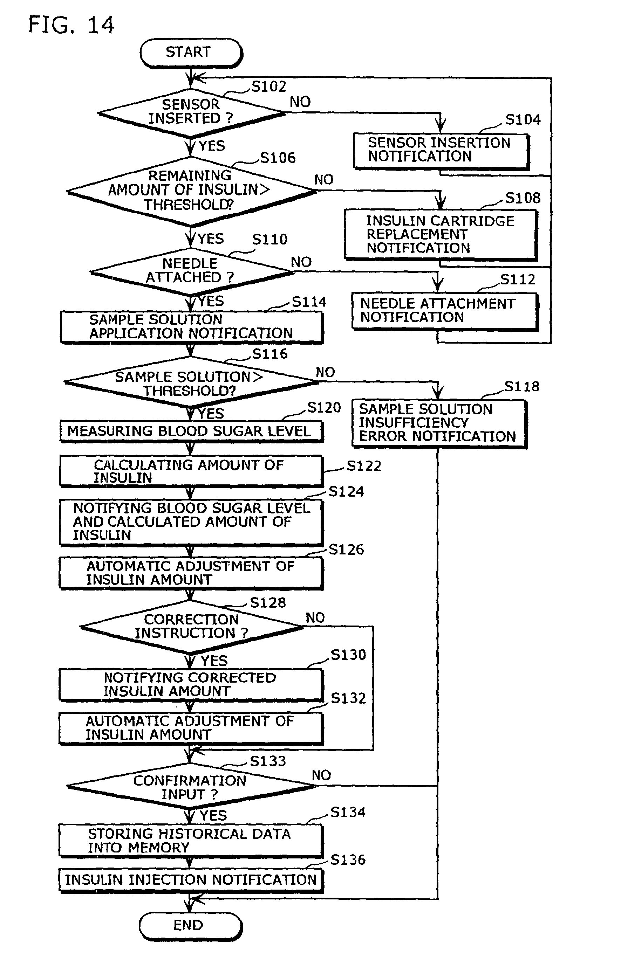

FIG. 14 is a flowchart showing a process flow of the syringe 110. The preparation the user makes prior to the blood sugar level measurement and the insulin injection is the same as that in the third embodiment. Also, processing from the sensor insertion judgment processing (S102) to the insulin amount calculation processing (S122) is the same as the processing from the sensor insertion judgment processing (S62) to the insulin amount calculation processing (S82) in the third embodiment explained with reference to FIG. 11. Therefore, the detailed explanation will not be repeated here.

After calculating the amount of insulin (S122), the CPU 50 notifies the blood sugar level and the calculated amount of insulin. At this time, the CPU 50 provides the set amount of insulin together with a confirmation message asking whether or not to consent to inject that amount of insulin (S124).

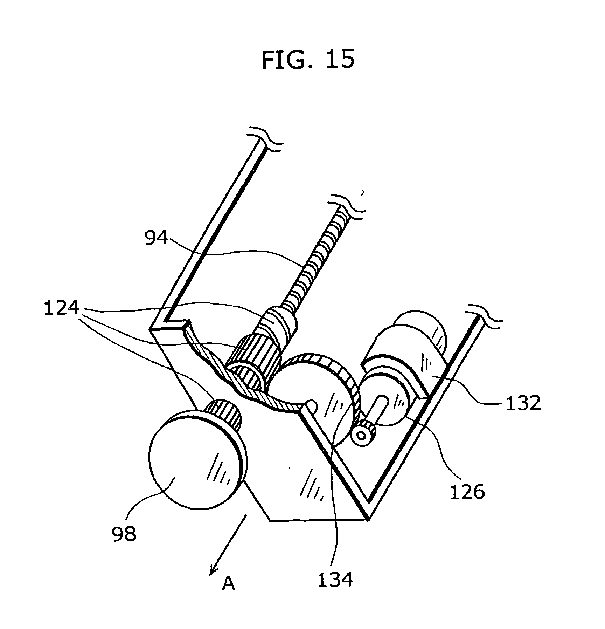

Next, the CPU 50 performs an automatic adjustment of the amount of insulin (S126). To be more specific, the CPU 50 and the motor 126 shown in FIG. 13 function as adjusting units, and adjust the position of the injection button 98 via the setting unit 124 in accordance with the calculated amount of insulin. The details are explained with reference to a rough schematic diagram in FIG. 15 showing the main parts of the adjusting unit of the present embodiment. Here, the motor 126 is fixed to the main body unit 112 by a motor supporting unit 132. First, in accordance with the calculated amount of insulin, the motor 126 is rotated under the control of the CPU 50. Next, the rotation of the motor 126 travels to the setting unit 124 through a gearing 134. According to the amount of this rotation, the setting unit 124 moves the injection button 98 in the direction of an arrow A in FIG. 15 (the direction opposed to the injecting direction in which the piston 94 discharges insulin into the injection needle 84). By means of this, it becomes possible for the injection button 98 to move the piston 94 in the injecting direction by this amount of movement. In this way, the amount of insulin to be injected is automatically set from the calculated amount of insulin.

The user may correct the set amount of insulin in consideration of the amounts of diet, exercise, etc as necessary. That is, the user increases or decreases the set amount of insulin by pushing the correction button 116a or 116b shown in FIG. 12. Then, the CPU 50 judges whether there is an instruction of an input of the amount of correction from the user (S128). It should be noted that whether or not there is an instruction of an input of the amount of correction is judged by whether or not a correction command is issued from the correction amount inputting unit 116 to the CPU 50 within a set period of time after the automatic adjustment processing was performed for the amount of insulin (S126).

If it is judged that there is a correction command for the amount of insulin (YES in S128), the CPU 50 notifies the user of the set amount of insulin that has been changed by the correction and also provides a confirmation message asking whether or not to consent to inject that amount of insulin (S130). Moreover, in accordance with the amount of insulin increased or decreased by correction, the automatic adjustment processing for the amount of insulin is performed again (S132).

When the automatic adjustment is made for the amount of insulin in accordance with the corrected amount of insulin (S132) or when it is judged that there is no correction command (NO in S128), the CPU 50 waits for a set period of time for the user to input, in response to the notification provided through the notification processing (S124 or S130), a confirmation of consenting to inject the insulin. If there is no such confirmation input (No in S133), the CPU 50 terminates the processing.

If there is such confirmation input (YES in S133), the CPU 50 stores the measured blood sugar level, the calculated amount of insulin, and the set amount of insulin together with at least the date or time of the measurement into the historical data storage area 48c (S134).

Finally, the CPU 50 notifies the user to inject the insulin (S136). In response to this notification, the user inserts the injection needle 84 into his/her body and then pushes the injection button 98. In consequence of this, the piston 94 discharges the insulin inside the insulin cartridge 90 into the injection needle 84. The discharged insulin is injected into the user's body through the injection needle 84.

Moreover, the sending/receiving processing for the historical data via the input/output terminal 38 is the same as that shown in FIGS. 5 and 6.

Moreover, it is possible to input data, such as an operation table, from the data inputting unit 36. This inputting method is the same as that presented in the third embodiment.

As explained up to this point, the present embodiment has operations and effects as described below in addition to the operations and effects in the above-mentioned embodiments.

That is, according to the present embodiment, the amount of insulin to be injected is automatically set in accordance with the calculated amount of insulin or the corrected amount of insulin. This allows the user whose vision has been deteriorated and the user whose function of fingers has deteriorated due to diabetic complication t o save the time and effort to manually set the amount of insulin, and to avoid a mistake in adjusting the amount of insulin to be injected.

In particular, since the correction amount inputting unit has such structure as to recognize the image of the buttons by touch, it is easier to manipulate for the user whose vision has deteriorated. Note that the projections and depressions of the correction amount inputting unit are not limited to the shapes of plus and minus signs and may be the shapes of arrows in the right and left directions, for example. Moreover, the correction amount inputting unit is not limited to buttons, and may be levers for increasing/decreasing the amount of correction by moving to the right and left (or back and forth), or a dial for increasing/decreasing the amount of correction by the amount of rotation, for example.

Moreover, the tolerance limits of dosage may be stored in the memory 48 beforehand, and when the corrected amount of insulin exceeds the tolerance limits, the CPU 50 may provide an alarm sound or a warning message.

### (Fifth Embodiment)

The following is an explanation about a health-care supporting system of the fifth embodiment of the present invention, with reference to the drawings.

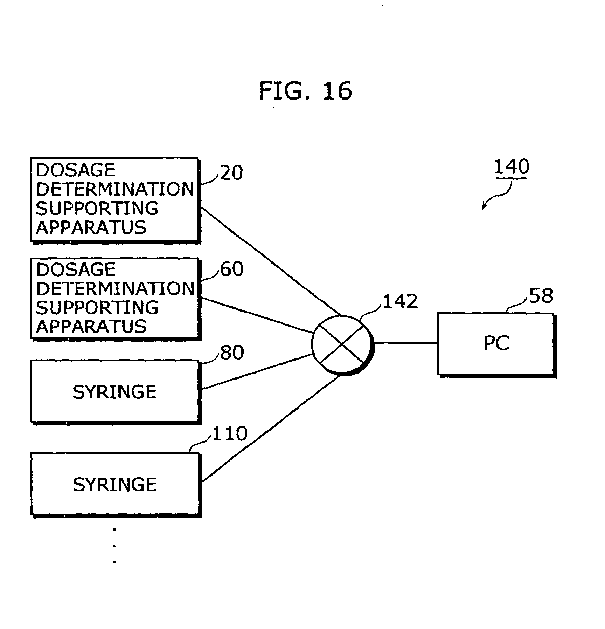

FIG. 16 is a block diagram showing the construction of the health-care supporting system of the fifth embodiment of the present invention. A health-care supporting system 140 is composed of a dosage determination supporting apparatus 20 used by a first patient, a dosage determination supporting apparatus 60 used by a second patient, a syringe 80 used by a third patient, a syringe 110 used by a fourth patient, and a PC 58 used by a physician in charge of the first to fourth patients, with the PC 58 being connected to the dosage determination supporting apparatus 20, the dosage determination supporting apparatus 60, the syringe 80, and the syringe 110 via a network 142.

A long-distance communication through the Internet etc. is possible for the respective communications between the PC 58 and the dosage determination supporting apparatus 20, the syringe 80, and the syringe 110. Meanwhile, for a communication between the dosage determination supporting apparatus 60 and the PC 58, only the short-distance wireless communication according to the Bluetooth or the like is possible as described above.

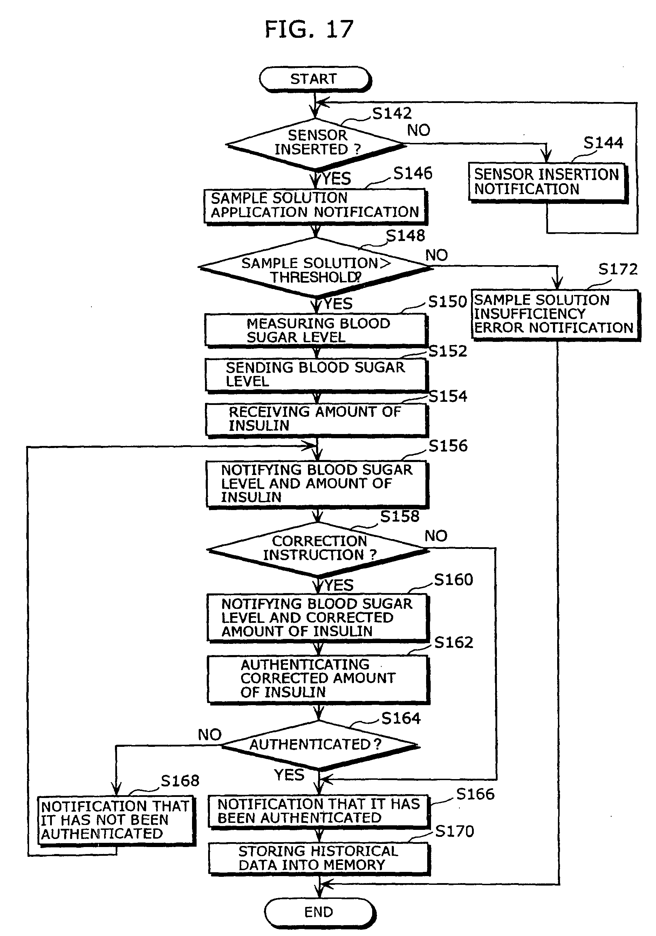

As one example, processing performed between the dosage determination supporting apparatus 20 and the PC 58 is explained with reference to FIGS. 17 to 19.

FIG. 17 is a flowchart of a process flow of the dosage determination supporting apparatus 20. Processing from the sensor insertion judgment processing (S142) to the blood sugar level measurement processing (S150) is the same as the processing from the sensor insertion judgment processing (S2) to the blood sugar level measurement processing (S10) of the dosage determination supporting apparatus 20 in the first embodiment explained with reference to FIG. 4.

After the blood sugar level measurement processing (S10) ends, the dosage determination supporting apparatus 20 transmits the measured blood sugar level to the PC 58 (S152), and receives the amount of insulin corresponding to the measured blood sugar level from the PC 58 (S154).

After receiving the amount of insulin, the CPU 50 of the dosage determination supporting apparatus 20 notifies the blood sugar level and the amount of insulin (S156).

After the notification processing (S156) is performed, the CPU 50 judges whether there is an instruction of an input of the amount of correction from the user (S158). Whether or not there is an instruction of an input of the amount of correction is judged by whether or not a correction command is issued from the data inputting unit 36 to the CPU 50 within a set period of time.

When the correction command is issued (YES in S158), the CPU notifies the blood sugar level and the corrected amount of insulin (S160). Then, the CPU 50 performs authentication processing in which it inquires the PC 58 as to whether the corrected amount of insulin is suitable (S162). According to the result of the authentication processing, the CPU 50 judges whether the corrected amount of insulin has been authenticated by the PC 58 (S164). When the corrected amount of insulin is authenticated by the PC 58 (YES in S164), a notification indicating that the authentication has been granted is provided (S166). After this, the measured blood sugar level, the corrected amount of insulin, and the measurement data/time are stored into the historical data storage area 48c of the memory 48 (S170).

If the corrected amount of insulin is not authenticated by the PC 58 (NO in S164), a notification indicating that it was not authenticated is provided (S186). Then, the notification processing for the blood sugar level and the amount of insulin is performed again (S156).

When the amount of blood is equal to or below the threshold (NO in S148), the amount of sample solution is judged to be insufficient. Then, a sample solution insufficiency error notification is provided (S172), where the measurement processing is terminated.

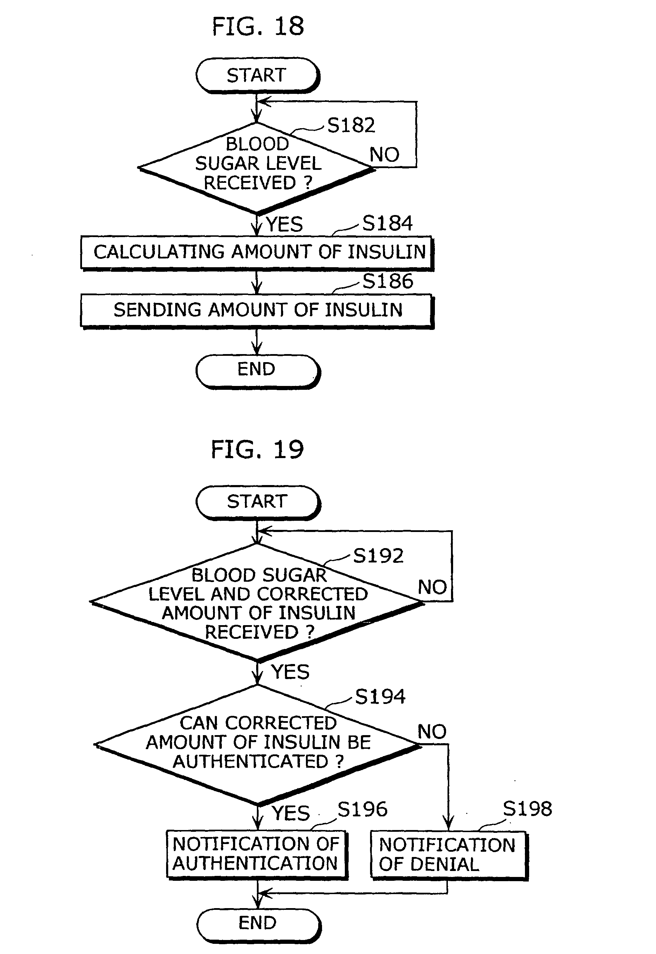

FIG. 18 is a flowchart of arithmetic processing for the amount of insulin performed by the PC 58. This processing corresponds to the blood sugar level sending processing (S152) and the insulin amount receiving processing (S154) shown in FIG 17.

The PC 58 waits to receive the blood sugar level from the dosage determination supporting apparatus 20 (S182). When receiving the blood sugar level (YES in S182), the PC 58 calculates the amount of insulin on the basis of the received blood sugar level. To be more specific, since the PC 58 stores the same operation tables as shown in FIG. 3, it calculates the amount of insulin from the blood sugar level and the receipt time of the blood sugar level (S184) and sends the amount of insulin to the dosage determination supporting apparatus 20 (S186).

FIG. 19 is a flowchart of authentication processing for the amount of insulin performed by the PC 58. This processing corresponds to the authentication processing (S162) for the corrected amount of insulin shown in FIG. 17.

The PC 58 waits to receive the blood sugar level and the corrected amount of insulin from the dosage determination supporting apparatus 20 (S192). When receiving the blood sugar level and the corrected amount of insulin (YES in S192), the PC 58 judges whether it is possible to authenticate the corrected amount of insulin (S194). The authentication possibility judgment processing (S194) is performed as follows, for example. Suppose that the PC 58 stores the blood sugar level and the corresponding tolerance limits of insulin dosage. The PC 58 judges whether it is possible to authenticate by judging whether the amount of insulin received from the dosage determination supporting apparatus 20 is within the tolerance limits concerned.

If the corrected amount of insulin is possible to be authenticated (YES in S194), the PC 58 sends an authentication notification to the dosage determination supporting apparatus 20 to indicate that it can be authenticated (S196). If the corrected amount of insulin is not possible to be authenticated (NO in S194), the PC 58 sends a denial notification to the dosage determination supporting apparatus 20 to indicate that the authentication has been denied (S198).

It should be noted that data sent/received in the processing performed between the dosage determination supporting apparatus 20 and the PC 58 shown in FIGS. 17 to 19 is also given a signature according to the public key cryptosystem so that the sender of the data is identified.

The dosage determination apparatus 60, the syringe 80, and syringe 110 may also inquire the PC 58 as to the authentication of the corrected amount of insulin, in addition to the processing in the stated embodiments. Moreover, the calculation of the amount of insulin may be performed by the PC 58.

As described up to this point, according to the present embodiment, a physician can manage physical conditions of a plurality of patients using one unit of PC 58. What is more, a signature is affixed to the data sent/received between the PC 58 and an apparatus used by a patient, according to the public key cryptosystem as explained above. Because of this, the physician can reliably know from which patient the received historical data has been sent.

Moreover, the patient can verify whether the received operation table is sent from his/her physician in charge.

Accordingly, the physician can provide a health-care supporting system that is able to determine a dosage with accuracy in accordance with a user's individual physical condition.

Moreover, the historical data is stored in both of the dosage determination supporting apparatus or syringe used by the user and the PC 58 used by the physician. On account of this, if a medical accident should happen, a quick after-the-fact action can be attained on the basis of the historical data.

Up to this point, although the dosage determination supporting apparatus, the syringe, and the health-care supporting system of the present invention are explained based on the embodiments, the present invention is not limited to these embodiments.

For example, the dosage determination supporting apparatus or the syringe in the above-mentioned embodiments has the construction where only either the long-distance communication or the short-distance wireless communication is possible. However, it should be understood that it may be provided with both of the communication functions.

Moreover, for the stated dosage determination supporting apparatus and syringe, the voltage is applied to the sensor and the blood sugar level is obtained on the basis of the current passing through the electrodes of the sensor. However, the method for measuring the blood sugar level is not limited to this. For example, an enzyme and a coloring matter that is discolored through enzyme reaction with a sample solution may be included in a reagent layer of the sensor, and the blood sugar level may be obtained by optically measuring the change in color of the coloring matter.

Furthermore, the method for calculating the amount of insulin is not limited to the stated embodiments. For example, using a table that shows a relation between the measurement time of the blood sugar level and the amount of insulin, the amount of insulin to be injected may be calculated from the time of day at which the blood sugar level was measured.

Moreover, the above-mentioned PC denotes a communication device or the like that can be connected not only to a PC, but also to a PDA (Personal Digital Assistant), a cellular phone, and a network. Also, a memory card of miniature size may be inserted into the input/output terminal 38 so that data can be inputted and outputted into/from it.

Furthermore, in the stated embodiments, the explanation has been given for a case where blood is used as a sample solution. However, it is not limited to this, and urine, saliva, etc. may be used as a sample solution.

Moreover, the explanation has been given for a case where glucose concentration is taken as an example of a characteristic of a sample solution. However, it is not limited to this, and hormone concentration, cholesterol concentration, etc. may be used as a characteristic of a sample solution.

Furthermore, insulin has been taken as an example of a medication to be injected. However, it is not limited to this, and may be a medication used for treating other diseases, a vitamin preparation, growth hormone, etc.

Moreover, the explanation has been given using the blood sugar level, the amount of insulin, the pulse, and the blood pressure as the historical data. However, a function by which other physical conditions can be obtained may be provided for the dosage determination supporting apparatus and the syringe. Then, body fat, bodily temperature, a pedometer may be measured, and these sets of measurement data may be used as historical data.

Moreover, although determination support for the dosage has been offered on the assumption that a human being is a patient in the present embodiment, it should be understood that he/she is not limited to a sick patient and may be a healthy person. Also, the present invention is applicable not only to human beings, but also to animals such as dogs and cats.

Furthermore, the explanation has been given on the assumption that the PC 58 is used by a physician. However, as long as it is legally satisfactory, a dentist, a pharmacist, a nurse, a hygienist, a dietitian, etc. other than a physician may use it.

Moreover, among the functions of the PC 58, a server that stores the historical data including the measured blood sugar level, the amount of insulin, the measurement day/time, the pulse, the blood pressure and so forth may be set in an information management center or the like outside the hospital.

Furthermore, besides original values obtained through the measurements or calculations, the stated various measurement results and dosages may include values that do not impair the purpose of the present invention even after predetermined conversion, transformation, modification, etc. were performed on them.

Moreover, an instrument called a lancet is usually used for blood sampling, and the lancet may be integral with the dosage determination supporting apparatus or the syringe.

By means of the present invention, a dosage determination supporting apparatus that is able to correctly determine the dosage in accordance with the physical condition of the user can be provided.

Also, a syringe that is able to correctly inject a dosage of medication set for the physical condition of the user can be provided.

Moreover, a health-care supporting system that is able to correctly determine a dosage in accordance with the physical condition of the user can be provided.

Furthermore, a communication method for the historical data and a communication method for the correspondence can be provided with a high degree of reliability.

In addition, history such as a dosage, biological information, a physical condition of the user can be used for health care.

Moreover, the user can always determine the optimal dosage in accordance with his/her physical condition.

Furthermore, the user can use the dosage determination supporting apparatus with a sense of security.

In addition, the user does not have to separately carry the dosage determination supporting apparatus and the syringe, and therefore the portability of the syringe improves. Also, the time taken from the dosage determination to the injection can be reduced.

Moreover, the incorrect amount of medication can be prevented from being administered.

Furthermore, the reliability of historical data regarding the physical condition of the user can be increased.

In addition, the reliability of the correspondence between the biological information and the dosage can be increased.

### Industrial Applicability

As described above, according to the present invention, a dosage in accordance with the physical condition of the user can be automatically obtained using the biological information extracted from the inside or surface of the user's body. In particular, even for a sight-impaired elderly person who has difficulty in obtaining a dosage by himself/herself and for a patient whose function of fingers has deteriorated, it becomes easy to obtain the dosage and thus the syringe that can automatically determine a dosage is suitable for them.

## Claims

1. A dosage determination supporting apparatus which supports a determination of a dosage of a medicine, comprising:
a measuring unit operable to measure biological information obtained from one of inside and surface of a user's body;
a dosage calculating unit operable to calculate a dosage on the basis of the biological information; and
a notifying unit operable to notify the user of the dosage calculated by the dosage calculating unit.

2. The dosage determination supporting apparatus according to Claim 1,
wherein the dosage calculating unit includes:
a correspondence memory operable to memorize a correspondence between the biological information and the dosage; and
a calculating unit operable to calculate the dosage corresponding to the biological information, with reference to the correspondence.

3. The dosage determination supporting apparatus according to Claim 2, further comprising:
a communication controlling unit, which is connected to an external terminal via a network, operable to control a communication with the external terminal; and
a correspondence rewriting unit operable to receive an external input via the communication controlling unit and to rewrite contents of the correspondence stored in the correspondence memory on the basis of the external input.

4. The dosage determination supporting apparatus according to Claim 3, further comprising
an external input authenticating unit operable to authenticate a person who inputs the external input,
wherein the correspondence rewriting unit rewrites the contents of the correspondence stored in the correspondence memory on the basis of the external input only when the person is approved.

5. The dosage determination supporting apparatus according to Claim 4,
wherein the external input authenticating unit performs an authentication on the basis of a physical characteristic of the person.

6. The dosage determination supporting apparatus according to Claim 2, further comprising:
a data inputting unit operable to receive an input of data from the user; and
a correspondence rewriting unit operable to receive an external input via the data inputting unit and to rewrite contents of the correspondence stored in the correspondence memory on the basis of the external input.

7. The dosage determination supporting apparatus according to Claim 1, further comprising:
a dosage memory operable to memorize the dosage; and
a dosage storing unit operable to store the calculated dosage together with a calculation time of day into the dosage memory.

8. The dosage determination supporting apparatus according to Claim 1, further comprising:
a biological information memory operable to memorize the biological information; and
a biological information storing unit operable to store the biological information measured by the measuring unit together with a measurement time of day into the biological information memory.

9. The dosage determination supporting apparatus according to Claim 1, further comprising:
a physical condition measuring unit operable to measure a physical condition of the user;
a physical condition memory operable to memorize the physical condition of the user; and
a physical condition storing unit operable to store the physical condition of the user together with a measurement time of day into the physical condition memory.

10. The dosage determination supporting apparatus according to Claim 1, further comprising:
a dosage memory operable to memorize the dosage;
a dosage storing unit operable to store the calculated dosage together with a calculation time of day into the dosage memory; and
a communication controlling unit, which is connected to an external terminal via a network, operable to output the dosage and the calculation time of day stored in the dosage memory to the external terminal via the network.

11. The dosage determination supporting apparatus according to Claim 1, further comprising:
a biological information memory operable to memorize the biological information;
a biological information storing unit operable to store the biological information measured by the measuring unit together with a measurement time of day into the biological information memory; and
a communication controlling unit, which is connected to an external terminal via a network, operable to output the biological information and the measurement time of day stored in the biological information memory to the external terminal via the network.

12. The dosage determination supporting apparatus according to Claim 1, further comprising:
a physical condition measuring unit operable to measure a physical condition of the user;
a physical condition memory operable to memorize a physical condition of the user;
a physical condition storing unit operable to store the physical condition of the user together with a measurement time of day into the physical condition memory; and
a communication controlling unit, which is connected to an external terminal via a network, operable to output the physical condition of the user and the measurement time of day stored in the physical condition memory to the external terminal via the network.

13. A syringe which is capable of setting a dosage automatically, comprising:
a measuring unit operable to measure biological information obtained from one of inside or surface of a user's body;
a dosage calculating unit operable to calculate a dosage on the basis of the biological information; and
an injecting unit operable to inject the dosage of a medicine calculated by the dosage calculating unit.

14. The syringe according to Claim 13,
wherein the dosage calculating unit includes:
a correspondence memory operable to memorize a correspondence between the biological information and the dosage; and
a calculating unit operable to calculate the dosage corresponding to the biological information, with reference to the correspondence.

15. The syringe according to Claim 14,
wherein the injecting unit includes:
an injection needle;
a medicine containing unit operable to contain the medicine;
a piston operable to discharge the medicine contained in the medicine containing unit into the injection needle;
a setting unit operable to set an amount of the medicine to be discharged by the piston; and
an adjusting unit operable to adjust the amount of the medicine to be discharged that is set by the setting unit so that the dosage of the medicine calculated by the calculating unit may be administered.

16. The syringe according to Claim 15 further comprising
a notifying unit operable to notify the user of the dosage calculated by the calculating unit.

17. The syringe according to Claim 16 further comprising
a user verification unit operable to have the user verify the dosage calculated by the calculating unit and to stop a discharge of the medicine when a verification is not obtained from the user.

18. The syringe according to Claim 15 further comprising
a correcting unit operable to correct the amount of the medicine to be discharged that is set by the setting unit.

19. The syringe according to Claim 18, further comprising:
a dosage storing memory operable to memorize the dosage; and
a dosage storing unit operable to store the dosage of the medicine having been administered to the user through the injecting unit together with an administration time of day into the dosage storing memory.

20. The syringe according to Claim 15, further comprising:
a dosage tolerance limits memory operable to memorize tolerance limits of the dosage; and
a discharged amount checking unit operable to check whether the amount of the medicine to be discharged is within the tolerance limits.

21. The syringe according to Claim 20 further comprising
a warning unit operable to warn the user when the amount of the medicine to be discharged is judged to be beyond the tolerance limits.

22. The syringe according to Claim 15, further comprising:
a dosage storing memory operable to memorize the dosage;
a dosage storing unit operable to store the dosage of the medicine having been administered to the user through the injecting unit together with an administration time of day into the dosage storing memory; and
a communication controlling unit, which is connected to an external terminal via a network, operable to output the dosage and the calculation time of day stored in the dosage storing memory to the external terminal via the network.

23. The syringe according to Claim 14, further comprising:
a communication controlling unit, which is connected to an external terminal via a network, operable to communicate with the external terminal; and
a correspondence rewriting unit operable to receive an external input via the communication controlling unit and to rewrite contents of the correspondence stored in the correspondence memory on the basis of the external input.

24. The syringe according to Claim 14, further comprising:
a data inputting unit operable to receive an input of data from the user; and
a correspondence rewriting unit operable to receive an external input via the data inputting unit and to rewrite contents of the correspondence stored in the correspondence memory on the basis of the external input.

25. The syringe according to Claim 13, further comprising:
a biological information memory operable to memorize the biological information; and
a biological information storing unit operable to store the biological information measured by the measuring unit together with a measurement time of day into the biological information memory.

26. The syringe according to Claim 13, further comprising:
a biological information memory operable to memorize the biological information;
a biological information storing unit operable to store the biological information measured by the measuring unit together with a measurement time of day into the biological information memory; and
a communication controlling unit, which is connected to an external terminal via a network, operable to output the biological information and the measurement time of day stored in the biological information memory to the external terminal via the network.

27. A health-care supporting system which supports a health care of a user, comprising:
a dosage determination supporting apparatus operable to support a determination of a dosage to be administered to the user, with reference to a correspondence between biological information obtained from one of inside or surface of the user's body and the dosage of a medicine; and
a server apparatus, which is connected to the dosage determination supporting apparatus via a network, operable to send the correspondence to the dosage determination supporting apparatus,
wherein the dosage determination supporting apparatus includes:
a measuring unit operable to measure the biological information of the user;
a correspondence memory operable to memorize the correspondence;
a calculating unit operable to calculate the dosage corresponding to the biological information, with reference to the correspondence;
a notifying unit operable to notify the user of the dosage calculated by the calculating unit; and
a correspondence rewriting unit operable to rewrite the correspondence that is received from the server apparatus and stored in the correspondence memory.

28. A server apparatus which is connected, via a network, to a dosage determination supporting apparatus that supports a determination of a dosage of a medicine to be administered to a user, and which sends/receives various kinds of data to/from the dosage determination supporting apparatus, comprising:
a correspondence memory operable to memorize a correspondence between biological information obtained from one of inside and surface of the user's body and the dosage;
a unit operable to receive the biological information from the dosage determination supporting apparatus;
a calculating unit operable to calculate the dosage corresponding to the received biological information, with reference to the correspondence memory; and
a unit operable to send the dosage calculated by the calculating unit to the dosage determination supporting apparatus.

29. A server apparatus which is connected, via a network, to a dosage determination supporting apparatus that supports a determination of a dosage of a medicine to be administered to a user, and which sends/receives various kinds of data to/from the dosage determination supporting apparatus, comprising:
a correspondence memory operable to memorize a correspondence between biological information obtained from one of inside and surface of a user's body and the dosage;
a unit operable to receive the biological information and the dosage from the dosage determination supporting apparatus;
a judging unit operable to judge, with reference to the correspondence memory, whether the dosage corresponding to the received biological information can be authenticated; and
a unit operable to send a judgment result given by the judging unit to the dosage determination supporting apparatus.

30. A method for historical-data communication according to a public key cryptosystem for a health-care supporting system which comprises a terminal apparatus used by a user and a server apparatus connected to the terminal apparatus and used by a health manager, comprising:
a step in which the terminal apparatus affixes a signature to the historical data regarding a health condition of the user, using a private key of the user;
a step in which the terminal apparatus sends the historical data having the affixed signature to the server apparatus;
a step in which the server apparatus receives the historical data having the affixed signature; and
a step in which the server apparatus verifies the signature affixed to the historical data, using a registered public key of the user.

31. A method for correspondence communication according to a public key cryptosystem for a health-care supporting system which comprises a dosage determination supporting apparatus used by a user and a server apparatus connected to the dosage determination supporting apparatus and used by a health manager,
wherein the correspondence shows a correspondence between biological information obtained from one of inside or surface of the user's body and a dosage, and
the dosage determination supporting apparatus obtains the dosage from the biological information, with reference to the correspondence,
the method comprising:
a step in which the server apparatus affixes a signature to the correspondence using a private key of the health manager;
a step in which the server apparatus sends the correspondence having the affixed signature to the dosage determination supporting apparatus; and
a step in which the dosage determination supporting apparatus receives the correspondence having the affixed signature; and
a step in which the dosage determination supporting apparatus verifies the signature affixed to the correspondence, using a registered public key of the health manager.
